# EUROPEAN PATENT SPECIFICATION

(11) **EP 1 506 771 B1**
(45) Date of publication and mention of the grant of the patent: **16.04.2008**
(21) Application number: 04018118.2
(22) Date of filing: 30.07.2004
(51) Int. Cl.: A61Q 11/00, A61Q 11/02, A61K 8/34, A61K 8/42, A61K 8/38

(54) **Gel-like tooth whitening material composition**
Zahnbleichmittel in Gelform
Gel à blanchissement pour les dents

(30) Priority: 04.08.2003 JP 2003285967
(43) Date of publication of application: 16.02.2005
(73) Proprietor: GC Corporation, Itabashi-ku, Tokyo (JP)
(72) Inventor: Mori, Daizaburo, Tokyo (JP); Yamaguchi, Shin, Tokyo (JP); Ikushima, Keisuke, Tokyo (JP)
(74) Representative: Müller-Boré & Partner Patentanwälte

(56) References cited:
- EP-A- 1 504 753
- WO-A-00/33792
- ES-A- 2 113 272
- US-A- 3 657 413
- US-A- 5 753 723
- DATABASE WPI Derwent Publications Ltd., London, GB; AN 2004-140515 XP002307581 EOM G A ET AL.: "Dry and thin film type delivery system for whitening teeth" & KR 2003 059 552 A (SK CHEM CO LTD) 10 July 2003 (2003-07-10)

## Description

The present invention relates to a gel-like tooth whitening material composition for whitening a pigmented tooth.

As whiteness of teeth is generally considered as an important cosmetic factor, there are strong demands for whitening teeth mainly in young females, and cases of requesting whitening of teeth are being increased. Whitening of teeth is basically to achromatize and/or remove a pigment deposited on a tooth through a chemical reaction, and such methods are mainly employed that use hydrogen peroxide, urea peroxide or titanium oxide. Examples of the methods include such a method in that a bleaching agent formed by mixing silicic anhydride of 30 to 35% or more and aqueous hydrogen peroxide is coated on a surface of a vital tooth to decompose aqueous hydrogen peroxide, and coloring substances are oxidized and decomposed through action of the decomposed products (as described, for example, in JP-A-5-320033), and such a method in that a redox reaction is caused with a photocatalyst, such as a titanium dioxide photocatalyst, todecolorize (bleach) a tooth (as described, for example, in JP-A-11-92351, JP-A-2000-344640, JP-A-2002-322041 and Japanese Patent No. 3,030,380), and bleaching methods obtained by combining these methods have also been known in the art.

A conventional thickener having been mainly used in a tooth whitening material using hydrogen peroxide, urea peroxide or titanium oxide is, for example, carboxypolymethylene, polyvinylpyrrolidone, hydroxyethyl cellulose or hydroxymethyl cellulose, as well as an inorganic mineral, such as saponite and magnesium sodium lithium silicate (as described, for example, in U.S. Patent No. 5, 631, 000 and U. S. Patent No. 6,500,408). However, in the case where these thickeners are used as a base agent, problems are caused at a time of filling a gel-like tooth whitening material into a tray by "adhesion" of the tooth whitening material to a spatula and a vessel, and "stringiness" of the gel occurring associated therewith. Furthermore, even in the case where the "adhesion" and the "stringiness" are to be avoided by changing the materials of the conventional thickener and adjusting the mixing proportions thereof, such problems occur in that the adhesiveness of the gel-like tooth whitening material to a tooth surface is lowered, and moreover, upon loading a tray into an oral cavity, the gel-like tooth whitening material outflows from the tray to the oral cavity. Accordingly, such a gel-like tooth whitening material is demanded that is improved in the aforementioned problems and has good operationality and good adhesiveness to a tooth surface.

The present invention is to solve the problem in operationality of a conventional gel-like tooth whitening material, and an object thereof is to provide such a gel-like tooth whitening material composition that does not cause the "adhesion" to a spatula or a vessel and the "stringiness", which are problems associated with the conventional gel-like tooth whitening material, is excellent in "adhesiveness to a tooth surface", and causes no "outflow from a tray".

As a result of earnest investigations made by the inventors for solving the problems, it has been found that all the problems can be solved by using a polyhydric alcohol as a solvent for a tooth whitening material composition, and simultaneously using a specific thickener.

The present invention relates to a gel-like tooth whitening material composition containing 60 to 90 parts by weight of a polyhydric alcohol as a primary solvent, 0.5 to 15 parts by weight of a methyl vinyl ether-maleic anhydride copolymer as a thickener, and 1 to 25 parts by weight of urea peroxide as a whitening component, the methyl vinyl ether-maleic anhydride copolymer being neutralized to a pH range of 5 to 7 under a condition containing no water.

It is preferred in the present invention that the methyl vinyl ether-maleic anhydride copolymer is a copolymer crosslinked with 1,9-decadiene.

It is also preferred in the present invention that the thickener contains the methyl vinyl ether-maleic anhydride copolymer in a proportion of 20% by weight or more.

It is also preferred in the present invention that the polyhydric alcohol is at least one selected from glycerin, diglycerin, propylene glycol, dipropylene glycol, sorbitol, mannitol, ethylene glycol, diethylene glycol; polyethylene glycol and polyethylene glycol monomethyl ether.

The gel-like tooth whitening material composition according to the present invention does not cause the "adhesion" to a spatula or a vessel and the "stringiness", which are problems associated with the conventional gel-like tooth whitening material, and is excellent in operationality of the gel and excellent in adhesiveness to a tooth surface.

The gel-like tooth whitening material composition according to the present invention is such a gel-like tooth whitening material composition that is used in the similar manner as the conventional tooth whitening material composition, which provides a whitening effect by leaving a whitening component, such as a peroxide, e.g., hydrogen peroxide and urea peroxide, and titanium oxide, on a tooth surface. The gel-like tooth whitening material composition contains 60 to 90 parts by weight of a polyhydric alcohol as a primary solvent, 0.5 to 15 parts by weight of a methyl vinyl ether-maleic anhydride copolymer as a thickener, and 1 to 25 parts by weight of urea peroxide as a whitening component, and the methyl vinyl ether-maleic anhydride copolymer is neutralized to a pH range of 5 to 7 under a condition containing no water. In preferred embodiments of the present invention, the methyl vinyl ether-maleic anhydride copolymer may be a copolymer crosslinked with 1,9-decadiene; the thickener may contain the methyl vinyl ether-maleic anhydride copolymer in a proportion of 20% by weight or more; and the polyhydric alcohol may be at least one selected from glycerin, diglycerin, propylene glycol, dipropylene glycol, sorbitol, mannitol, ethylene glycol, diethylene glycol, polyethylene glycol and polyethylene glycol monomethyl ether.

The polyhydric alcohol used as a primary solvent in the gel-like tooth whitening material composition according to the present invention can make the tooth bleaching material composition as a gel having high operationality by combining with the methyl vinyl ether-maleic anhydride copolymer described later. As the polyhydric alcohol, any polyhydric alcohol may be used that has been used in a tooth paste and an oral composition, and in particular, glycerin, polyglycerin, such as diglycerin, propylene glycol, dipropylene glycol, sorbitol, mannitol, ethylene glycol, diethylene glycol, polyethylene glycol and polyethylene glycol monomethyl ether are preferred from the standpoint of safety.

The polyhydric alcohol used in the present invention is mixed in the tooth whitening material composition in an amount of 60 to 90 parts by weight. In the case where the amount is less than 60 parts by weight, the effect thereof is difficult to be obtained, and in the case where it exceeds 90 parts by weight, there is such a possibility that the whitening effect is impaired.

The methyl vinyl ether-maleic anhydride copolymer used as a thickener in the present invention is neutralized to a pH range of 5 to 7 under a condition containing no water, and provides, by combining with the aforementioned polyhydric alcohol, such a effect that has not been obtained by the conventional thickener, i.e., upon filling the gel-like tooth whitening material composition in a tray, it does not cause the "adhesion" to a spatula or a vessel, the "stringiness" and the "outflow after coating", but provides an effect of improving the operationality of the gel and simultaneously improves the adhesiveness to a tooth surface.

In general, a methyl vinyl ether-maleic anhydride copolymer is a water soluble polymer that is soluble in both water and an organic solvent, and it is widely used in such purposes as a cohesive layer of various kinds of tape formulations, a thickening agent of tooth paste, and a coating agent, by utilizing ring-opening of anhydride groups due to reaction with water or alcohol. The methyl vinyl ether-maleic anhydride copolymer may be used in the present invention irrespective of the polymerization method thereof. In order to improve the operationality and the adhesiveness of the tooth whitening material composition to a tooth surface, which is a characteristic feature of the present invention, it is preferred that the proportion of the methyl vinyl ether-maleic anhydride copolymer mixed in the thickener is 20% by weight or more. Furthermore, it is also preferred in view of stability in viscosity against heat etc. that the methyl vinyl ether-maleic anhydride copolymer is crosslinked with 1,9-decadiene.

It is necessary that the composition of present invention has a pH range of 5 to 7. So the methyl vinyl ether-maleic anhydride copolymer is necessary to be neutralized. At this time, neutralizing agent used herein is not particularly limited as far as it partially or totally neutralizes carboxyl groups of the methyl vinyl ether-maleic anhydride copolymer, and preferred examples of the neutralizing agent include sodium hydroxide, potassium hydroxide, ammonium hydroxide, monoethanolamine, diethanolamine and triethanolamine. It is important that no water as solvent is used when neutralizing the methyl vinyl ether-maleic anhydride copolymer with the neutralizing agent, and it is preferred that the copolymer is neutralized with propylene glycol, which is a kind of the aforementioned polyhydric alcohol. Alkalization due to an excess amount of the neutralizing agent is not preferred at this time, and therefore, the pH thereof is 7 at most.

The methyl vinyl ether-maleic anhydride copolymer used in the present invention is mixed in the tooth whitening material composition in an amount of 0.5 to 15 parts by weight. In the case where the amount is less than 0.5 part by weight, the effect of the addition thereof is difficult to be obtained, and in the case where it exceeds 15 parts by weight, there is such a possibility that the effect of the addition thereof is impaired.

The methyl vinyl ether-maleic anhydride copolymer used in the present invention is a water soluble polymer that is soluble in both water and an organic solvent, and therefore, upon contacting with a water content in an oral cavity during the use of the gel-like tooth whitening material composition, it swells with water, in addition to the polyhydric alcohol, to thicken the tooth whitening material composition. Accordingly, by utilizing such an effect, the whitening component, such as urea peroxide, can be prevented from being eluted even in the case where the tooth whitening material composition is diluted with saliva or the like in the oral cavity. Therefore, in order to improve the thickening effect after contacting with water in an oral cavity, the gel-like tooth whitening material composition characteristically does not contain water in advance.

The whitening component used in the present invention is urea peroxide, which is hard to be alkalized upon containing water. Urea peroxide has such a problem in that it is liable to be decomposed with water to lower the stability thereof. Therefore, the gel-like tooth whitening material composition of the present invention is made as a tooth whitening material composition containing no water as described in the foregoing, so as to improve significantly the storage stability of urea peroxide as the whitening component, which is started to be decomposed with water.

The mixing amount of urea peroxide is 1 to 25 parts by weight in the tooth whitening material composition. In the case where the amount is less than 1 part by weight, the whitening effect is difficult to be obtained, and in the case where it exceeds 25 parts by weight, there is such a tendency that the operationality is impaired.

The gel-like tooth whitening material composition according to the present invention may further contain 0.1 to 10 parts by weight of an inorganic thickener and a conventional organic thickener per 100 parts by weight in total of the polyhydric alcohol as a primary solvent, the methyl vinyl ether-maleic anhydride copolymer as a thickener, and urea peroxide as a whitening component, for the purpose of fine adjustment of viscosity. Examples of the inorganic thickener include those having been used in tooth whitening materials, and specific examples thereof include magnesium sodium silicate, calcium carbonate, calcium silicate, magnesium silicate, silica powder, various kinds of glass, amorphous hydrous silicic acid and fumed silica. Examples of the organic thickener include carboxypolymethylene, polyvinylpyrrolidone, hydroxyethyl cellulose and hydroxymethyl cellulose.

The gel-like tooth whitening material composition according to the present invention may further contain, in addition to the aforementioned components, a flavor, a coloring material, various kinds of stabilizers, and a solvent containing substantially no water.

### EXAMPLES

The present invention will be described in more detail with reference to the following examples, but the invention is not construed as being limited thereto.

### Production of Tooth Whitening Material Composition

As shown in Table 1 below, two or more kinds selected from polyethylene glycol (weight average molecular weight: 400), glycerin and propylene glycol as primary solvents were agitated, to which urea peroxide as the whitening component and the methyl vinyl ether-maleic anhydride copolymer as the thickener were dispersed. Furthermore, one kind or two kinds selected from magnesium sodium silicate powder, several kinds of silica powder (Aerosil A200, Aerosil R972 and Aerosil OX50, trade names, produced by Nippon Aerosil Co. , Ltd.) and titanium dioxide as the inorganic thickener, polyvinylpyrrolidone and carboxypolymethylene (Carbopol, a trade name, produced by NIKKO CHEMICAL Co. , Ltd.) as the organic thickener, a flavor (Tooth Paste Flavor, a trade name, produced by Takasago International Corp.), and sodium fluoride and potassium nitrate for reinforcing dentin were added depending on necessity, so as to produce gel-like tooth whitening material compositions. The methyl vinyl ether-maleic anhydride copolymer was crosslinked with 1,9-decadiene, and was neutralized to a pH of 5 to 7 before mixing with one of propylene glycol solution having potassium hydroxide, sodium hydroxide or a propylene glycol (concentration of 5% by weight).

### Evaluation of Operationality

(1) A tray for whitening was produced so as to accommodate with teeth of a subject.
(2) The gel-like tooth whitening material compositions of the examples and the comparative examples shown in Table 1 were filled in the tray, and the evaluation with respect to the "adhesion to a spatula" was made based on the following standard.

AA: When filling in the tray, the gel was not adhered to a spatula to provide good operationality.
A: When filling in the tray, the gel was slightly adhered to a spatula, but substantially no problem occurred in operationality.
B: Substantially no problem occurred in.
C: When filling in the tray, the gel was adhered to a spatula to cause adverse affects on operationality.

The results obtained are collectively shown in Table 1.

### Evaluation of Adhesiveness to Tooth Surface

(1) A tray for whitening was produced so as to accommodate with teeth of a subject.
(2) The gel-like tooth whitening material compositions of the examples and the comparative examples shown in Table 1 were filled in the tray, and the tray was fit on teeth in an oral cavity.
(3) The state after lapsing two hours after fitting was observed, and the evaluation of the adhesiveness on a tooth surface was made based on the following standard.

AA: No outflow of the tooth whitening material composition from the tray was observed.
A: The tooth whitening material composition was suppressed from outflowing by absorbing saliva to a certain extent.
B: Some outflow of the tooth whitening material composition from the tray was observed.
C: A large amount of outflow of the tooth whitening material composition due to saliva was observed.

The results obtained are collectively shown in Table 1.

It is understood from the results shown in Table 1 that the gel-like tooth whitening material composition according to the present invention is excellent in operationality and adhesiveness on a tooth surface. * Examples beyond scope of claim 1

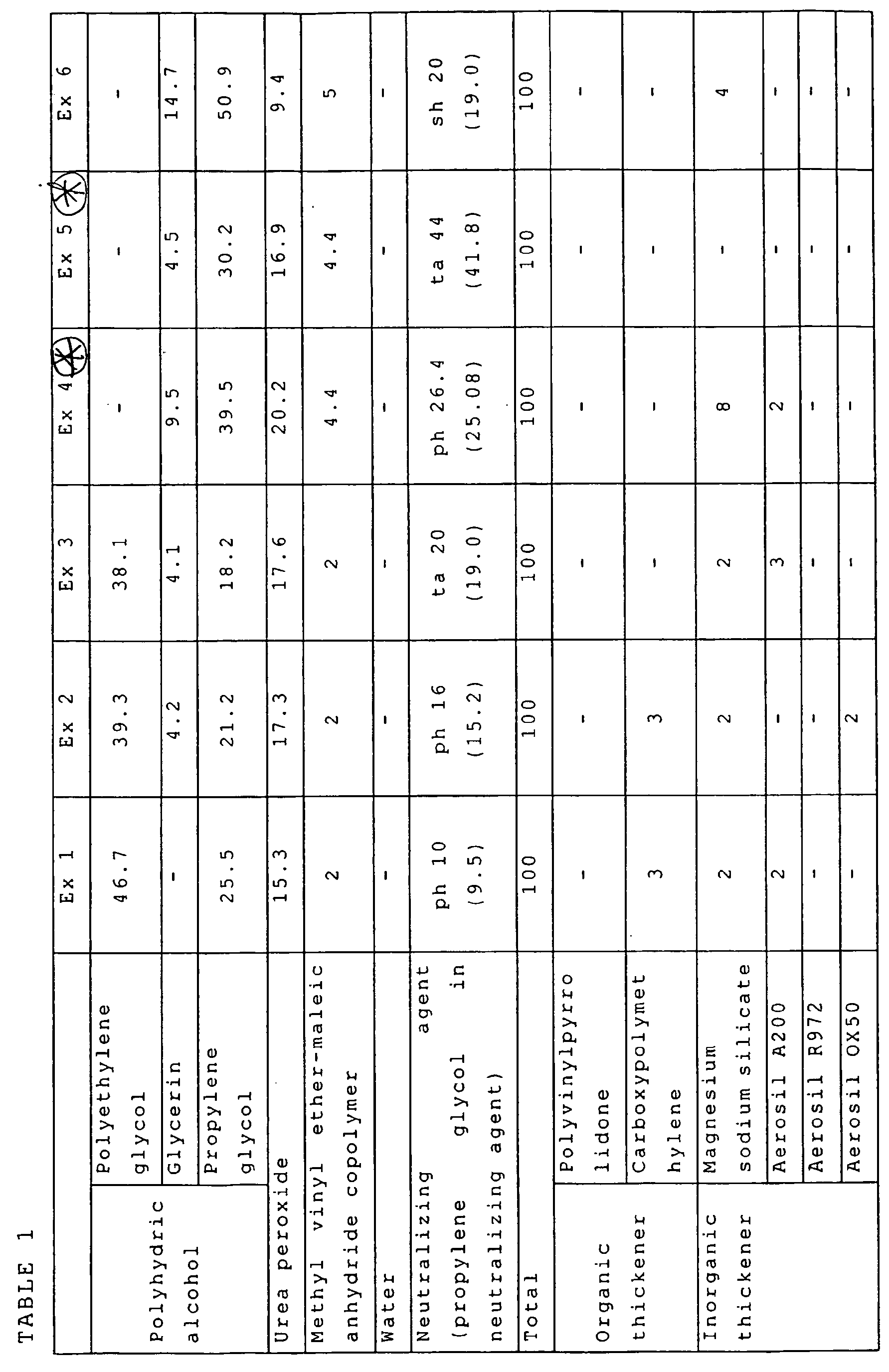

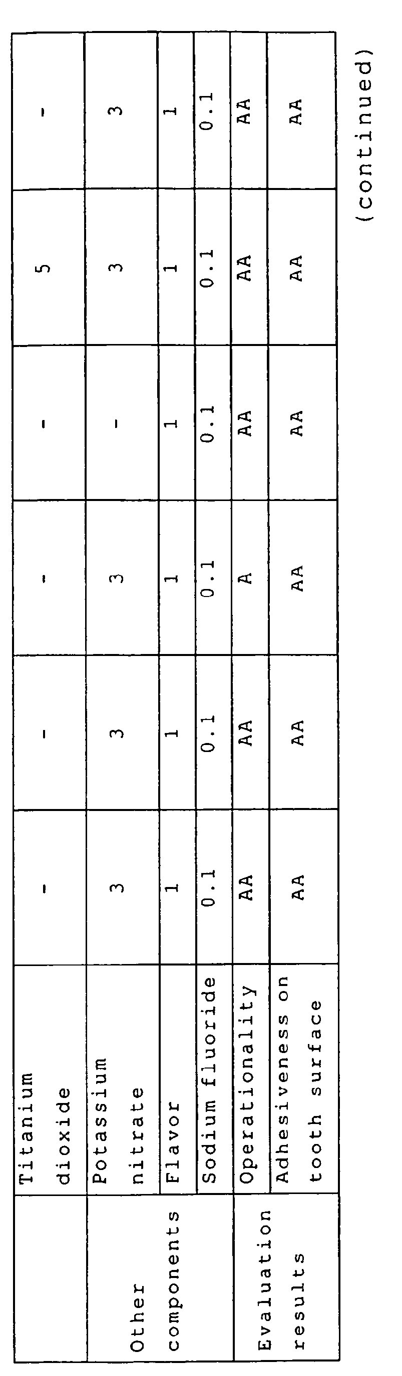

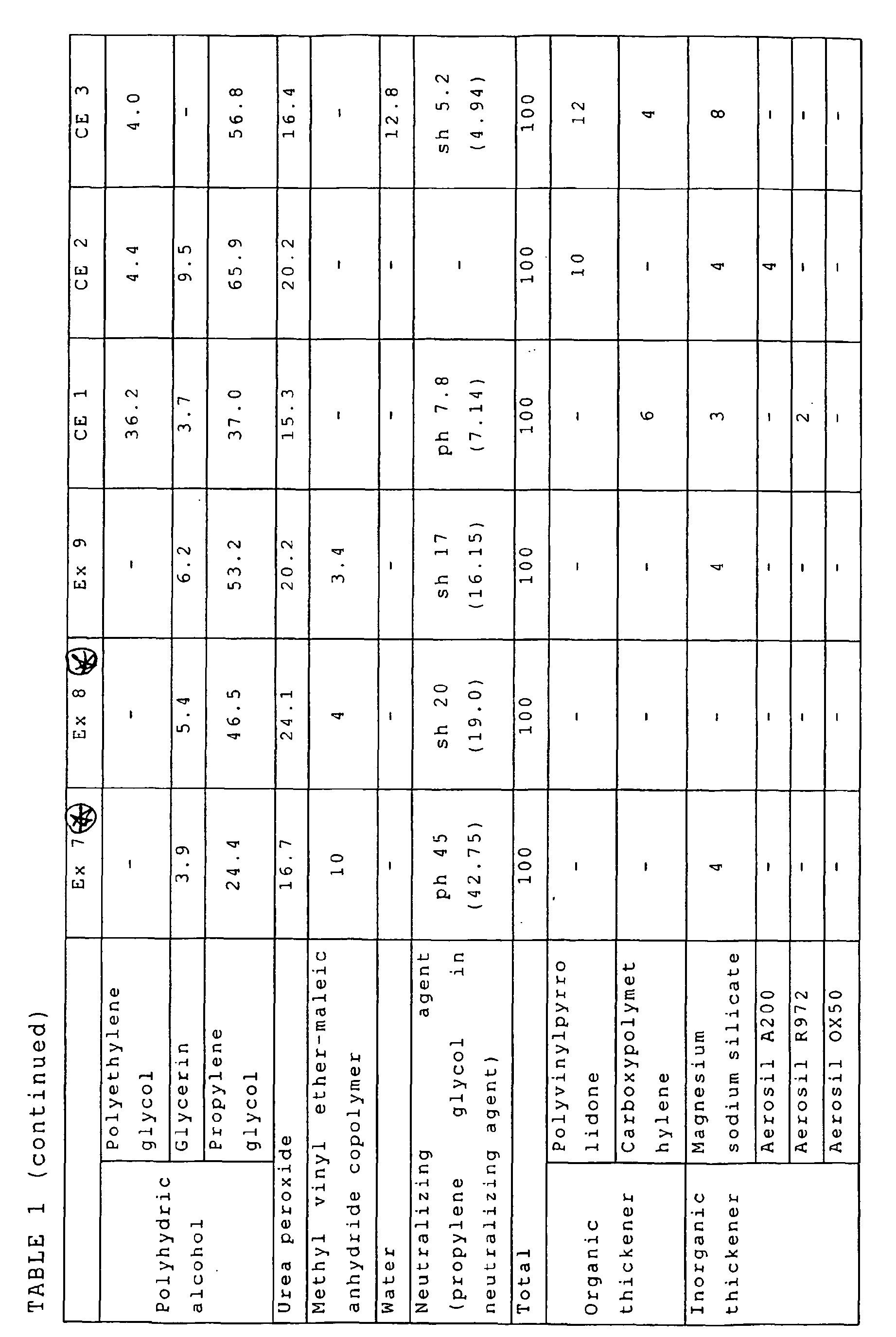

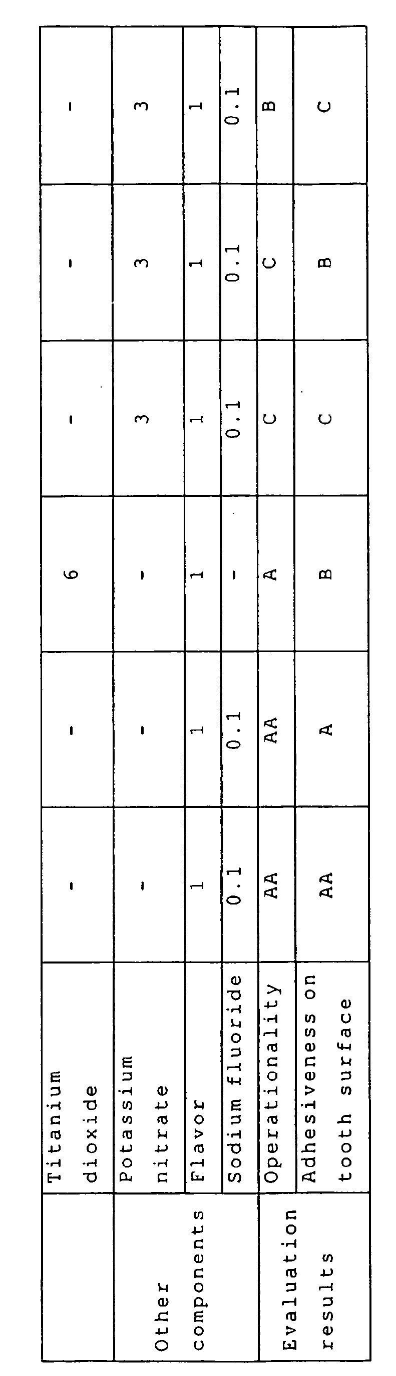

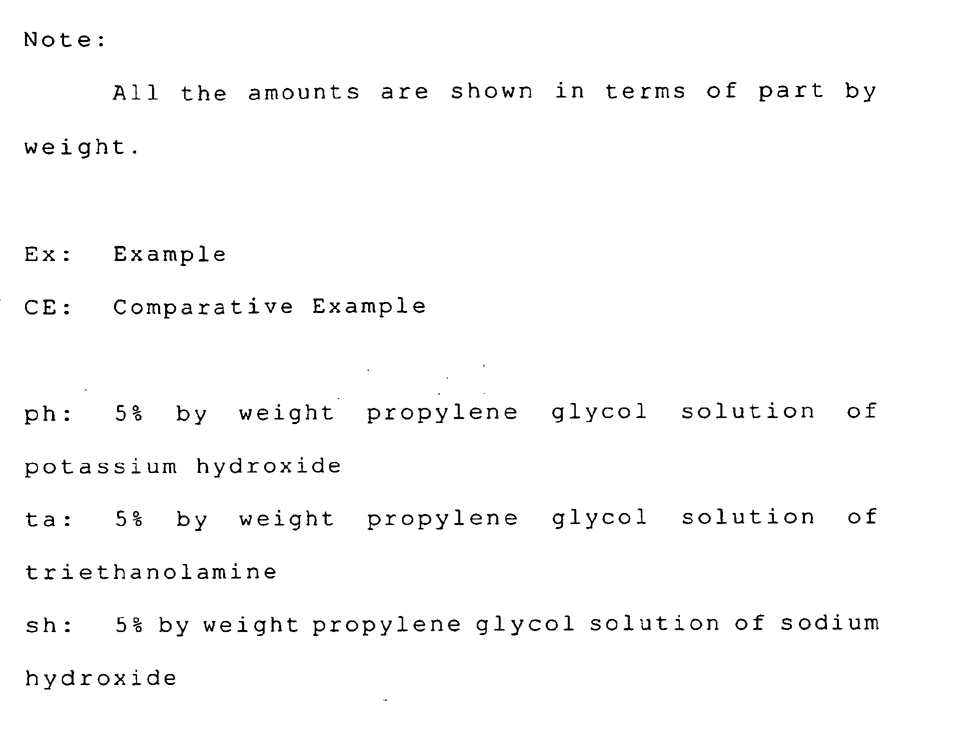

## Claims

1. A gel-like tooth whitening material composition comprising 60 to 90 parts by weight of a polyhydric alcohol as a primary solvent, 0. 5 to 15 parts by weight of a methyl vinyl ether-maleic anhydride copolymer as a thickener, and 1 to 25 parts by weight of urea peroxide as a whitening component, the methyl vinyl ether-maleic anhydride copolymer being neutralized to a pH range of 5 to 7 under a condition containing no water.

2. A gel-like tooth whitening material composition as claimed in claim 1, wherein the methyl vinyl ether-maleic anhydride copolymer is a copolymer crosslinked with 1,9-decadiene.

3. A gel-like tooth whitening material composition as claimed in claim 1 or 2, wherein the thickener contains the methyl vinyl ether-maleic anhydride copolymer in a proportion of 20% by weight or more.

4. A gel-like tooth whitening material composition as claimed in any one of claims 1 to 3, wherein the polyhydric alcohol is at least one selected from glycerin, diglycerin, propylene glycol, dipropylene glycol, sorbitol, mannitol, ethylene glycol, diethylene glycol and polyethylene glycol, polyethylene glycol monomethyl ether.

## Patentansprüche

1. Gel-ähnliche Zahnweißmaterialzusammensetzung, umfassend 60 bis 90 Gew.-Teile eines mehrwertigen Alkohols als ein Hauptlösungsmittel, 0,5 bis 15 Gew.-Teile eines Methylvinylether-Maleinsäureanhydrid-Copolymers als ein Verdickungsmittel und 1 bis 25 Gew.-Teile eines Harnstoffperoxids als eine Weißfärbungskomponente, wobei das Methylvinylether-Maleinsäureanhydrid-Copolymer auf einen pH-Bereich von 5 bis 7 unter einer Bedingung, enthaltend kein Wasser, neutralisiert worden ist.

2. Gel-ähnliche Zahnweißmaterialzusammensetzung, wie in Anspruch 1 beansprucht, wobei das Methylvinylether-Maleinsäureanhydrid-Copolymer ein mit 1,9-Decadien vernetztes Copolymer ist.

3. Gel-ähnliche Zahnweißmaterialzusammensetzung, wie in Anspruch 1 oder 2 beansprucht, wobei das Verdickungsmittel das Methylvinylether-Maleinsäureanhydrid-Copolymer in einem Anteil von 20 Gew.-% oder mehr enthält.

4. Gel-ähnliche Zahnweißmaterialzusammensetzung, wie in einem der Ansprüche 1 bis 3 beansprucht, wobei der mehrwertige Alkohol mindestens einer, ausgewählt aus Glycerin, Diglycerin, Propylenglykol, Dipropylenglykol, Sorbitol, Mannitol, Ethylenglykol, Diethylenglykol und Polyethylenglykol, Polyethylenglykolmonomethylether, ausgewählt ist.

## Revendications

1. Composition de matière pour le blanchiment des dents ressemblant à un gel, comprenant 60 à 90 parties en poids d'un polyol en tant que solvant primaire, 0,5 à 15 parties en poids d'un copolymère d'éther méthylvinylique-anhydride maléique en tant qu'épaississant et 1 à 25 parties en poids de peroxyde d'urée en tant que composant de blanchiment, le copolymère d'éther méthylvinylique-anhydride maléique étant neutralisé dans une plage de pH de 5 à 7 dans des conditions ne contenant pas d'eau.

2. Composition de matière pour le blanchiment des dents ressemblant à un gel selon la revendication 1, dans laquelle le copolymère d'éther méthylvinylique-anhydride maléique est un copolymère réticulé avec du 1,9-décadiène.

3. Composition de matière pour le blanchiment des dents ressemblant à un gel selon la revendication 1 ou 2, dans laquelle l'épaississant contient le copolymère d'éther méthylvinylique-anhydride maléique dans une proportion de 20 % en poids ou plus.

4. Composition de matière pour le blanchiment des dents ressemblant à un gel selon l'une quelconque des revendications 1 à 3, dans laquelle le polyol est au moins un élément choisi parmi le glycérol, le diglycérol, le propylèneglycol, le dipropylèneglycol, le sorbitol, le mannitol, l'éthylèneglycol, le diéthylèneglycol, le polyéthylèneglycol et l'éther monométhylique de polyéthylèneglycol.
